Europäisches Patentamt

European Patent Office

Office européen des brevets

(11) Publication number: **0 047 986**
**B1**

(12) **EUROPEAN PATENT SPECIFICATION**

(45) Date of publication of patent specification: **27.12.85**

(51) Int. Cl.⁴: **A 61 B 5/02**

(21) Application number: **81107140.6**

(22) Date of filing: **10.09.81**

(54) Integral hermetic implantable pressure transducer.

(30) Priority: **12.09.80 US 186373**

(43) Date of publication of application:
**24.03.82 Bulletin 82/12**

(45) Publication of the grant of the patent:
**27.12.85 Bulletin 85/52**

(84) Designated Contracting States:
**DE FR GB NL SE**

(56) References cited:
**FR-A-2 420 210**
**GB-A-2 031 592**
**US-A-2 796 863**
**US-A-3 038 465**
**US-A-3 811 427**
**US-A-3 811 429**

(73) Proprietor: **MEDTRONIC, INC.**
**3055 Old Highway Eight**
**Minneapolis Minnesota 55440 (US)**

(72) Inventor: **Anderson, Kenneth M.**
**9125 West Bush Lake Road**
**Bloomington Minnesota 55438 (US)**

(74) Representative: **Schwan, Gerhard, Dipl.-Ing.**
**Elfenstrasse 32**
**D-8000 München 83 (DE)**

Courier Press, Leamington Spa, England.

## Description

The present invention relates to an implantable pressure transducer for measuring pressure within the human heart, comprising an elongated, flexible lead body of a material substantially inert to body fluid, having a proximal end and a distal end; an outer chamber filled with a first fluid whereby relative pressure of the first fluid is determined by relative ambient pressure external to the outer chamber; a basic pressure transducer mounted within the outer chamber, which basic pressure transducer has electrical interface leads and defines an inner chamber filled with a second fluid; a vent aperture for venting the inner chamber, whereby pressure of the first fluid relative to pressure of the vent aperture may be electrically sensed from electrical changes at the interface leads; conductors electrically coupled to the interface leads; and a vent passage provided in the lead body and filled with the second fluid, the vent passage having a first end sealably coupled to the vent aperture and a second end located a sufficient distance from the vent aperture such that when the outer chamber is located within a ventrical of the human heart, the second end of the vent passage is located external to the heart.

Such an implantable pressure transducer is known from US—A—3 811 427. In the case of this known pressure transducer the outer chamber is defined by the distal end of the lead body which must be flexible to allow its introduction into the heart through a vein or an artery. The proximal part of the lead body defines the vent passage which communicates with outside air. The pressure transducer is connected by wires to a measuring device without further particulars being disclosed.

In conformity with the present invention
— the outer chamber is provided in a metal cylinder of a pressure transducer head fixedly mounted to the distal end of the lead body;
— the vent passage is defined by a vent tube extending within the lead body;
— a membrane is sealably coupled to the second end of the vent passage and is exposed to the exterior of the lead body in a desired body cavity, whereby pressure in this body cavity is transmitted to the second fluid;
— the outer chamber as well as the inner chamber and the vent passage communicating with the latter are vacuum filled with the first and second fluids, respectively; and
— the conductors extend through the lead body and are terminated at the proximal end of the lead body by connectors.

In fact, a differential pressure transducer comprising a semiconductor pressure sensor mounted in a stainless steel housing; a high pressure seal diaphragm separating one of the surfaces of the pressure sensor from a space in which a high-pressure fluid is introduced; a high-pressure side sealed liquid filled in a space defined by the high-pressure seal diaphragm and the one surface of the pressure sensor; a low-pressure seal diaphragm separating the other surface of pressure sensor from a space in which a low pressure fluid is introduced; and a low pressure side sealed liquid filled in a space defined by said low-pressure seal diaphragm and said other surface of said semiconductor pressure sensor, is known from GB—A—2 031 592. This pressure transducer, however, is not designed for use within the human body.

In a preferred embodiment of the invention described in detail below a monolithic, silicon-based, piezoresistive semiconductor element is used as the basic pressure transducer although a piezocapacitive element could also be used. The basic pressure transducer is attached to a glass substrate via adhesive. The electrical conductors pass through the glass substrate and are wire bonded to the basic pressure transducer. The glass substrate is welded to seal one end of a titanium cylinder. Other metals such as stainless steel could be used. Titanium is preferred because it does not corrode in the body. A very thin diaphragm of titanium is welded to the titanium cylinder to seal the other end. The chamber thus created, which contains the basic pressure transducer, is vacuum filled with oil. Pressure of a fluid impinging upon the titanium diaphragm is transmitted to the basic pressure transducer via the oil.

The titanium diaphragm is protected from damage by a titanium grill, which also assists in insertion for transvenous application. Since it does not act as a hermetic seal, the grill could also be a polymer like urethane. Tines, loops, or other additions may be made to aid in anchoring the assembly in the desired position.

The basic pressure transducer is electrically coupled via the conductors to implantable electrode circuitry. The inner chamber of the basic pressure transducer is vented to an area, such as the abdominal cavity.

One way of carrying out the invention is described in detail below with reference to drawings which illustrate only one specific embodiment.

Fig. 1 is a schematic view of the preferred embodiment of the entire pressure transducer.

Fig. 2 is an electrical equivalency diagram of the pressure transducer.

Fig. 3 is a schematic view of a typical application of the present pressure transducer.

Fig. 4 is a side sectional view of the pressure transducer head.

Fig. 5 is a top view of the protecting grill.

Fig. 6 is a side sectional view of the protecting grill.

Fig. 7 is a top view of the titanium diaphragm.

Fig. 8 is a side sectional view of the titanium diaphragm.

Fig. 9 is a bottom view of glass substrate.

Fig. 10 is a top view of case bottom.

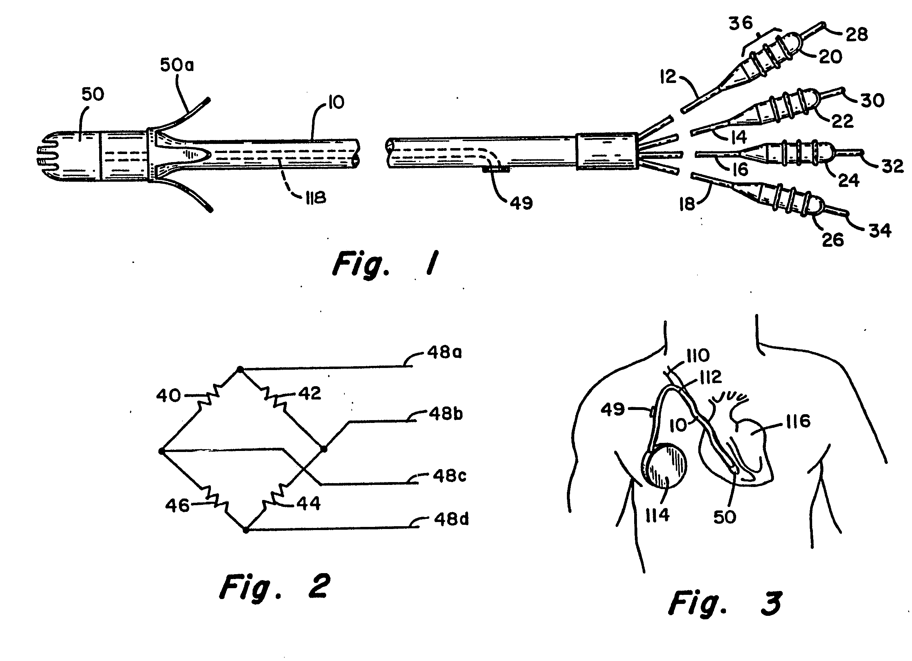

As shown in Fig. 1 a pressure transducer head 50 is attached to a lead body 10. Lead body 10 contains the electrical conductors which couple the pressure transducer head 50 to the associated electronic circuitry. The conductors are coils to reduce bending stress and, thus, decrease flex breakage. Lead body 10 terminates in the four conductors 12, 14, 16 and 18 each of which is terminated by an electrical connector body (i.e., 20, 22, 24 and 26 respectively) with an associated connection pin (i.e., 28, 30, 32 and 34, respectively). In keeping with the practices in the fabrication of implantable pacing leads for chronic application, lead body 10 and the four conductors 12, 14, 16 and 18, are coated with a material substantially inert to body fluids such as silicon rubber or urethane. Connector bodies 20, 22, 27 and 26 also contain O-rings 36 for sealing the electrical connections against ingress of body fluids. Connector body 10 is sealed to pressure transducer head 50 by the injection molding of silicon rubber.

Fig. 2 shows the electrical characteristics of the pressure transducer. Notice that electrically the circuit is a resistive bridge represented by fixed resistors 40, 42, 44 and 46. Each point of the bridge is connected to a different one of interface leads 48a, 48b, 48c and 48d. As the measured pressure changes, the relative resistance of the resistive bridge legs change which is sensed via interface leads 48a, 48b, 48c and 48d.

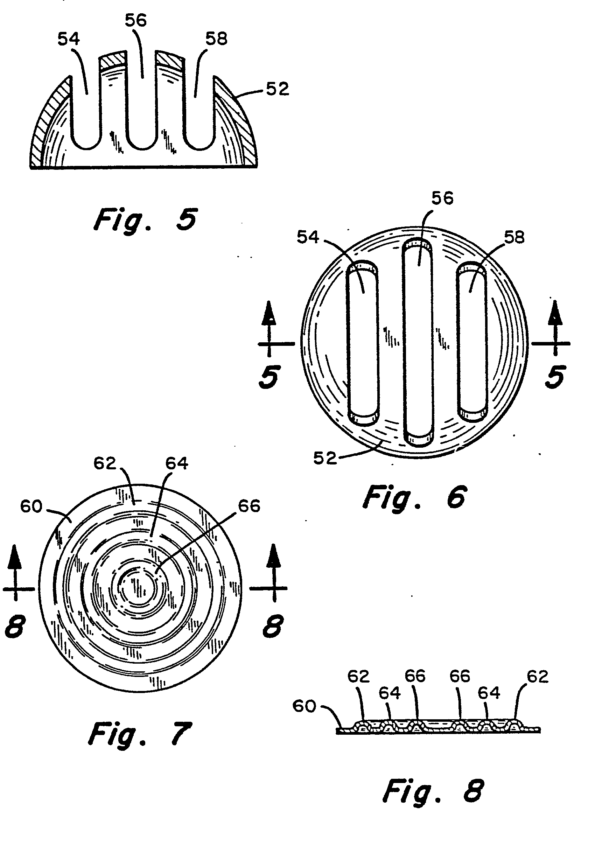

Fig. 4 is a side sectional view of the pressure transducer head 50. A basic pressure transducer 72 is mounted internal to the pressure transducer head 50. The basic pressure transducer 72 is available commercially. US—A—4 023 562 shows the construction and operation of a suitable basic pressure transducer 72, although electrical coupling is made via wire bonding in the present invention and not via the chip holder used in the reference. The basic pressure transducer is comprised of upper silicon diaphragm 72a which flexes with pressure changes, inner chamber 72b, silicon substrate 72c, and hermetic bond 72d. Basic pressure transducer 72 is adhesively bonded to a glass substrate 80. A glass having nearly the same temperature coefficient of expansion as silicon is chosen, such as Corning 7070. Four lead-in conductors of which only two, namely 92, 96, are shown in Fig. 4, are electrically coupled to basic pressure transducer 72 via common wire bonds 76. Each of the lead-in conductors is electrically coupled to a different one of four separate conductors located within lead body 10 and thence to one of connection pins 28, 30, 32 and 34 (see also Fig. 1).

Glass substrate 80 is adhesively attached to a pressure transducer base 82 which may be made of titanium, ceramic, or other suitable material. Glass is presently used because using an insulator obviates the need to insulate the feedthrough holes for the lead-in conductors. The glass base is encased in a header 68 which is a cylinder of titanium.

Header 68 is inserted into a titanium outer cylinder 70. The outer cylinder is sealed about its inner circumference to header 68 by weld 78. A titanium diaphragm 60 seals the opposite end to outer cylinder 70. Weld 74 seals the entire outer circumference and also attaches a grill 52, provided the grill is metal and not polymer.

The inner chamber 72b of basic pressure transducer 72 is vented. To accomplish this an aperture 100 is created in the silicon substrate 72c which is coupled to a tube 84, coupling inner chamber 72b to tube 84. Tube 84 is coupled to a corresponding tube 118 within lead body 10. Tube 118 is vented via a silastic membrane 49. Inner chamber 72b, tube 84 and tube 118 are vacuum filled with oil through tube 104 drilled through base 82 and glass substrate 80. This oil transmits pressure variations from silastic membrane 49 to inner chamber 72b and silicon diaphragm 72a via tubes 118 and 84, thus permitting differential pressure measurements. Plug 106 is used to seal tube 104. In fabricating the device, care must be exercised in creating seal 102 between the silicon substrate 72c of basic pressure transducer 72 and glass substrate 80.

Body fluid into which pressure transducer head 50 is immersed, freely enters grill 52 via slots 54, 56 and 58 impinging upon diaphragm 60. The pressure of that body fluid is transmitted by the oil within the outer chamber to basic pressure transducer 72. That pressure is measured against the pressure of the inner chamber 72b by flexing of silicon diaphragm 72a. This flexing results in electrical changes measured as resistive changes in the resistive bridge circuit of Fig. 2.

Fig. 6 is a top view of grill 52 with slots 54, 56 and 58 visible. Fig. 5 provides a side sectional view of grill 52. The present material used is titanium although stainless steel or a polymer like urethane would also seem feasible. The principal purpose of grill 52 is protection of diaphragm 60, and to aid in sliding down an artery or vein. This protection is most critical during implantation, although chronic protection is also required because of anticipated movement. Grill 52 must have openings to permit impingement of body fluid upon diaphragm 60. It is important that these openings permit smooth flow of body fluids to ensure cleaning of diaphragm 60 of any solid material such as small blood clots. The present design uses slots 54, 56 and 58, although other shapes are also acceptable.

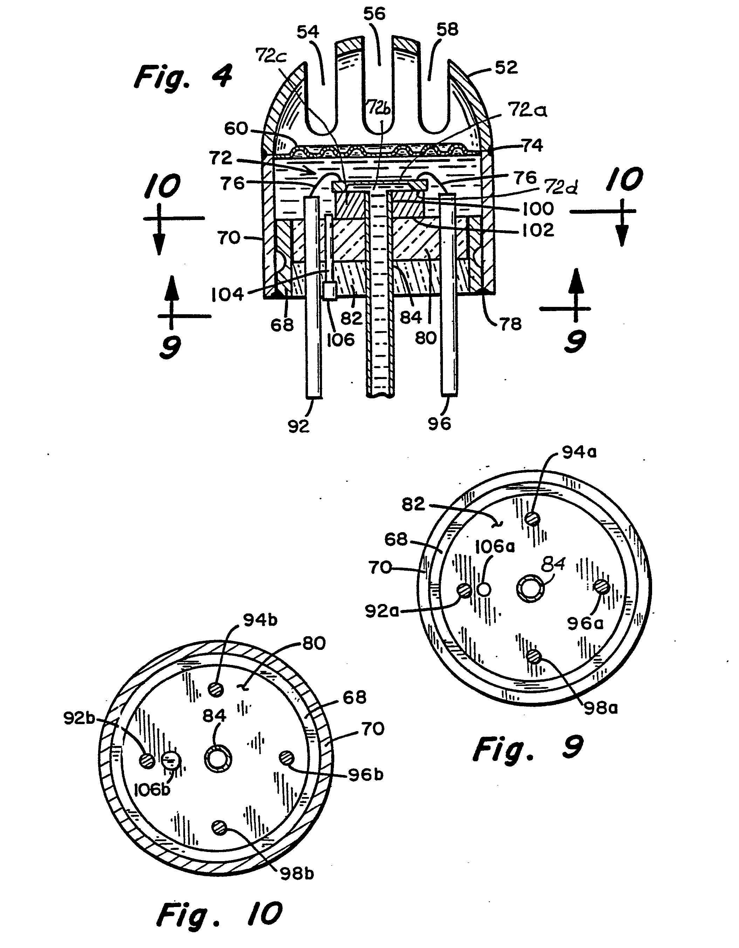

Fig. 7 is a top view of diaphragm 60. It is a titanium disc having a diameter of about 6.35 mm (one fourth inch) having a thickness of 0.025 to 0.051 mm (1—2 mils). It is important that a material be used which is sufficiently thin and flexible to properly transmit the pressure changes of the bodily fluid while having sufficient tensile strength to provide a chronic seal. To increase the compliance (flexibility) of diaphragm 60, ridges 62, 64 and 66 are stamped into the disc as shown. The side sectional view provided in Fig. 8 shows ridges 62, 64 and 66 which are about 0.13 mm (five one-thousandths of an inch) in height.

Fig. 9 is a bottom sectional view of pressure transducer head 50 showing pressure transducer base 82. As explained above, use of glass or other

insulator for pressure transducer base 82 obviates the need to insulate feedthrough holes 92a, 94a, 96a, and 98a for the four lead-in conductors. Aperture 106a permits the outer chamber to be oil-filled.

Fig. 10 is a top sectional view of pressure transducer head 50 showing glass substrate 80. The outer chamber is oil-filled via aperture 106b. Feedthrough holes 92b, 94b, 96b and 98b are sealed after insertion of the lead-in conductors.

Fig. 3 shows a typical application for the present pressure transducer. An incision is made permitting access to vein 110. Incision 112 is made in vein 110. Pressure transducer head 50 is inserted into heart 116 via vein 110. The remainder of lead body 10 extends from incision 112 to implantable electronic circuit 114 and is electrically coupled thereto. In the present embodiment, the pressure of blood within the right ventrical of heart 116 relative to the pressure within a desired body cavity, such as the abdominal cavity, may thus be sensed by implantable electronic circuit 114. Tines 50a or other position maintaining structures may be arranged appurtenant to pressure transducer head 50 in accordance with techniques for transvenous implantation of pacing leads.

**Claims**

1. An implantable pressure transducer for measuring pressure within the human heart, comprising
an elongated, flexible lead body (10) of a material substantially inert to body fluid, having a proximal end and a distal end;
an outer chamber filled with a first fluid whereby relative pressure of said first fluid is determined by relative ambient pressure external to said outer chamber; a basic pressure transducer (72) mounted within said outer chamber, which basic pressure transducer has electrical interface leads (48a, 48b, 48c, 48d) and defines an inner chamber (72b) filled with a second fluid;
a vent aperture (100) for venting said inner chamber (72b), whereby pressure of said first fluid relative to pressure of said vent aperture may be electrically sensed from electrical changes at said interface leads (48a, 48b, 48c, 48d);
conductors (12, 14, 16, 18) electrically coupled to said interface leads (48a, 48b, 48c, 48d); and
a vent passage provided in said lead body and filled with the second fluid, said vent passage having a first end sealably coupled to said vent aperture (100) and a second end located a sufficient distance from said vent aperture such that when said outer chamber is located within a ventricle of the human heart, said second end of said vent passage is located external to said heart, characterized in that,
— said outer chamber is provided in a metal cylinder (70) of a pressure transducer head (50) fixedly mounted to the distal end of said lead body (10);

— said vent passage is defined by a vent tube (118) extending within said lead body (10);
— a membrane (49) is sealably coupled to said second end of said vent passage and is exposed to the exterior of said lead body (10) in a desired body cavity, whereby pressure in said body cavity is transmitted to said second fluid;
— said outer chamber as well as said inner chamber (72b) and the vent passage communicating with the latter are vacuum filled with said first and second fluids, respectively; and
— said conductors (12, 14, 16, 18) extend through said lead body (10) and are terminated at said proximal end of said lead body by connectors (20, 22, 24, 26).

2. An implantable pressure transducer according to claim 1 wherein said outer chamber comprises a base structure (68, 80, 82) of rigid material sealably attached to a first end of the rigid cylindrical sides of the metal cylinder (70), and a top defined by a diaphragm (60) sealably attached to a second end of said cylinder sides whereby ambient pressure external to said outer chamber is essentially transmitted through said top to said first fluid.

3. An implantable pressure transducer according to claim 1 or 2 wherein said first fluid and said second fluid are oil.

4. An implantable pressure transducer according to claim 2 or 3 wherein said basic pressure transducer (72) is a semiconductor device fixedly mounted to a substrate (80) of said base structure (68, 80, 82).

5. An implantable pressure transducer according to claim 4 wherein said substrate (80) is glass having a temperature coefficient of expansion similar to said semiconductor device.

6. An implantable pressure transducer according to claim 4 or 5 wherein said semiconductor device is of silicon.

7. An implantable pressure transducer according to any one of claims 2 to 6 wherein said cylinder sides and said top are titanium.

8. An implantable pressure transducer according to any one of claims 2 to 7 further comprising a grill (52) having at least one aperture permitting ingress and egress of body fluids fixedly mounted to said second end of said cylinder sides thereby covering said top.

9. An implantable pressure transducer according to claim 8 wherein said grill (52) is titanium.

**Revendications**

1. Transducteur de pression implantable destiné à mesurer la pression dans le coeur humain, comportant un corps de conducteur (10) souple et allongé d'une matière substantiellement inerte aux fluides du corps, comprenant une extrémité proximale et une extrémité distale; une chambre extérieure remplie d'un premier fluide de manière qu'une pression relative dudit premier fluide soit déterminée par rapport à une pression ambiante extérieure à ladite chambre extérieure, un transducteur de pression de base (72) monté dans ladite chambre extérieure, ce transducteur de pression de base comportant des conducteurs électriques d'interface (48a, 48b, 48c, 48d) et définissant une chambre intérieure (72b) remplie avec un second fluide; une ouverture de communication (10) destinée à mettre en communication avec l'extérieur ladite chambre intérieure (72b) de manière qu'une pression dudit premier fluide par rapport à la pression de ladite ouverture de communication puisse être détectée électriquement par des variations électriques sur lesdits conducteurs d'interface (48a, 48b, 48c, 48d); des conducteurs (12, 14, 16, 18) couplés électriquement avec lesdits conducteurs d'interface (48a, 48b, 48c, 48d); et un passage de communication prévu dans ledit corps de conducteur et rempli avec le second fluide, ledit passage de communication comprenant une première extrémité couplée de façon étanche avec ladite ouverture de communication (100) et une seconde extrémité située à une distance suffisante de ladite ouverture de communication pour que, lorsque la chambre extérieure est située dans un ventricule du corps humain, ladite seconde extrémité dudit passage de communication se trouve à l'extérieur dudit coeur, caractérisé en ce que ladite chambre extérieure est prévue dans un cylindre métallique (70) d'une tête de transducteur de pression (50) montée de façon fixe sur l'extrémité distale dudit corps de conducteur (10); ledit passage de communication est défini par un tube de communication (118) passant dans ledit corps de conducteur (10); une membrane (49) est couplée de façon hermétique avec la seconde extrémité dudit passage de communication et elle est exposée à l'extérieur dudit corps de conducteur (10) dans une cavité voulue du corps, de manière que la pression dans ladite cavité du corps soit transmise audit second fluide; ladite chambre extérieure ainsi que ladite chambre intérieure (72b) et le passage de communication communiquant avec cette dernière sont remplis sous vide avec ledit premier et ledit second fluide respectivement; et lesdits conducteurs (12, 14, 16, 18) passent par ledit corps de conducteur (10) et se terminent à ladite extrémité proximale dudit corps de conducteur par des connecteurs (20, 22, 24, 26).

2. Transducteur de pression implantable selon la revendication 1, dans lequel ladite chambre extérieure comporte une structure de base (68, 80, 82) d'une matière rigide fixée de façon étanche sur une première extrémité des côtés cylindriques rigides du cylindre métalique (70) et un dessus défini par un diaphragme (60) fixé hermétiquement sur une seconde extrémité desdits côtés du cylindre de manière qu'une pression ambiante extérieure à ladite chambre extérieure soit essentiellement transmise par ledit dessus vers ledit premier fluide.

3. Transducteur de pression implantable selon la revendication 1 ou 2, dans lequel ledit premier fluide et ledit second fluide sont de l'huile.

4. Transducteur de pression implantable selon la revendication 2 ou 3, dans lequel ledit transducteur de pression de base (72) est un composant à semiconducteurs monté de façon fixe sur un substrat (80) de ladite structure de base (68, 80, 82).

5. Transducteur de pression implantable selon la revendication 4, dans lequel ledit substrat (80) est en verre ayant un coefficient de dilatation thermique similaire à celui dudit composant semi-conducteur.

6. Transducteur de pression implantable selon la revendication 4 ou 5, dans lequel ledit composant semiconducteur est en silicium.

7. Transducteur de pression implantable selon l'une quelconque des revendications 2 à 6, dans lequel lesdits côtés du cylindre et ledit dessus sont en titane.

8. Transducteur de pression implantable selon l'une quelconque des revendications 2 à 7, comprenant en outre une grille (52) avec au moins une ouverture permettant l'entrée et la sortie de fluides du corps, montée de façon fixe sur ladite seconde extrémité desdits côtés dudit cylindre, couvrant ainsi ledit dessus.

9. Transducteur de pression implantable selon la revendication 8, dans lequel ladite grille (52) est en titane.

**Patentansprüche**

1. Implantierbarer Druckwandler zur Messung des Drucks im menschlichen Herzen mit

einem langgestreckten flexiblen Leitungskörper (10) aus einem gegenüber Körpermedien im wesentlichen inerten Werkstoff, der ein proximales und ein distales Ende aufweist;

einer mit einem ersten Fluid gefüllten Außenkammer, wodurch der relative Druck des ersten Fluids durch den ausserhalb der Außenkammer herrschenden relativen Außendruck bestimmt wird;

einem innerhalb der Außenkammer angeordneten Basisdruckwandler (72), der elektrische Verbindungsleitungen (48a, 48b, 48c, 48d) aufweist und eine mit einem zweiten Fluid gefüllte Innenkammer (72b) bildet;

einer Druckausgleichsöffnung (100) zum Herstellen einer Verbindung mit der Innenkammer (72b), wodurch der Druck des ersten Fluids gegenüber dem Druck der Druckausgleichsöffnung anhand von elektrischen Änderungen an den Verbindungsleitungen (48a, 48b, 48c, 48d) elektrisch erfaßt werden kann;

Leitern (12, 14, 16, 18), die mit den Verbindungsleitungen (48a, 48b, 48c, 48d) elektrisch gekoppelt sind; und einem in dem Leitungskörper vorgesehenen und mit dem zweiten Fluid gefüllten Druckausgleichskanal, der ein mit der Druckausgleichsöffnung (100) in dichtender Verbindung stehendes erstes Ende und ein zweites Ende aufweist, das in ausreichendem Abstand von der Druckausgleichsöffnung angeordnet ist, so daß dann, wenn sich die Außenkammer innerhalb eines Ventrikels des menschlichen Herzens befindet, das zweite Ende des Druckausgleichskanals außerhalb des Herzens liegt,

dadurch gekennzeichnet, daß

— die Außenkammer in einem Metallzylinder (70) eines Druckwandlerkopfs (50) vorgesehen ist, der an dem distalen Ende des Leitungskörpers (10) fest angebracht ist;

— der Druckausgleichskanal von einem Druckausgleichsrohr (118) gebildet wird, das innerhalb des Leitungskörpers (10) verläuft;

— eine Membran (49) mit dem zweiten Ende des Druckausgleichskanals, dichtend gekoppelt und dem Äußeren des Leitungskörpers (10) in einer gewünschten Körperhöhle ausgesetzt ist, wodurch der in der Körperhöhle herrschende Druck auf das zweite Fluid übertragen wird;

— die Außenkammer ebenso wie die Innenkammer (72b) und der mit Letzterer in Verbindung stehende Druckausgleichskanal im Vakuum mit dem ersten bzw. zweiten Fluid gefüllt sind; und

— die Leiter (12, 14, 16, 18) durch den Leitungskörper (10) hindurchreichen und an dem proximalen Ende des Leitungskörpers in Anschlüssen (20, 22, 24, 26) enden.

2. Implantierbarer Druckwandler nach Anspruch 1, wobei die Außenkammer eine Basisanordnung (68, 80, 82) aus starrem Werkstoff, die mit einem ersten Ende der starren zylindrischen Seitenwände des Metallzylinders (70) dichtend verbunden ist, und eine Oberseite aufweist, die von einer Membran (60) gebildet ist, die an einem zweiten Ende der Zylinderseitenwände dichtend angebracht ist, wodurch der außerhalb der Außenkammer herrschende Druck im wesentlichen über die Oberseite auf das erste Fluid übertragen wird.

3. Implantierbarer Druckwandler nach Anspruch 1 oder 2, wobei das erste Fluid und das zweite Fluid Öl sind.

4. Implantierbarer Druckwandler nach Anspruch 2 oder 3, wobei der Basisdruckwandler (72) eine Halbleiteranordnung ist, die an einem Substrat (80) der Basisanordnung (68, 80, 82) fest angebracht ist.

5. Implantierbarer Druckwandler nach Anspruch 4, wobei das Substrat (80) aus Glas mit einem Wärmedehnungskoeffizienten besteht, der ähnlich demjenigen der Halbleiteranordnung ist.

6. Implantierbarer Druckwandler nach Anspruch 4 oder 5, wobei die Halbleiteranordnung aus Silizium besteht.

7. Implantierbarer Druckwandler nach einem der Ansprüche 2 bis 6, wobei die Zylinderseitenwände und die Oberseite aus Titan bestehen.

8. Implantierbarer Druckwandler nach einem der Ansprüche 2 bis 7 mit einem Grill (52), der mindestens eine den Eintritt und Austritt von Körpermedien erlaubende Öffnung aufweist, an dem zweiten Ende der Zylinderseitenwände fest angebracht ist und auf diese Weise die Oberseite überdeckt.

9. Implantierbarer Druckwandler nach Anspruch 8, wobei der Grill (52) aus Titan besteht.

Fig. 1

Fig. 2

Fig. 3

Fig. 5

Fig. 6

Fig. 7

Fig. 8

Fig. 4

Fig. 9

Fig. 10